# EUROPÄISCHE PATENTSCHRIFT

(11) **EP 0 927 184 B1**
(45) Veröffentlichungstag und Bekanntmachung des Hinweises auf die Patenterteilung: **22.10.2003**
(21) Anmeldenummer: 97940118.9
(22) Anmeldetag: 22.08.1997
(51) Int. Cl.: C07D 495/14, A61K 31/435

(54) **3-SUBSTITUIERTE PYRIDO 4',3':4,5] THIENO 2,3-D]PYRIMIDIN-DERIVATE, IHRE HERSTELLUNG UND VERWENDUNG**
3-SUBSTITUTED PYRIDO 4',3':4,5]THIENO 2,3-D]PYRIMIDINE DERIVATIVES, THEIR PREPARATION AND THEIR USE
DERIVES PYRIDO 4',3':4,5]THIENO 2,3-D]PYRIMIDINE SUBSTITUES EN POSITION 3, LEUR PREPARATION ET LEUR UTILISATION

(30) Priorität: 10.09.1996 DE 19636769
(43) Veröffentlichungstag der Anmeldung: 07.07.1999
(73) Patentinhaber: ABBOTT GmbH & Co. KG, 65205 Wiesbaden (DE)
(72) Erfinder: STEINER, Gerd, D-67281 Kirchheim (DE); LUBISCH, Wilfried, D-68159 Mannheim (DE); BACH, Alfred, D-69123 Heidelberg (DE); EMLING, Franz, D-67065 Ludwigshafen (DE); WICKE, Karsten, D-67112 Altrip (DE); TESCHENDORF, Hans-Jürgen, D-67373 Dudenhofen (DE); BEHL, Berthold, D-67117 Limburgerhof (DE); KERRRIGAN, Frank, Nottingham NG1 1GF (GB); CHEETHAM, Sharon, Nottingham NG1 1GF (GB)
(74) Vertreter: Grünecker, Kinkeldey, Stockmair & Schwanhäusser Anwaltssozietät
(86) Internationale Anmeldenummer: EP9704593
(87) Internationale Veröffentlichungsnummer: WO98011110

(56) Entgegenhaltungen:
- EP-A- 0 329 168
- US-A- 4 835 157

## Beschreibung

Die Erfindung betrifft neue 3-substituierte Pyrido [4',3':4,5] thieno [2,3-d] pyrimidin-Derivate, deren Herstellung und Verwendung zur Herstellung von Pharmawirkstoffen.

Die klassischen Antidepressiva und auch die neueren selektiven Serotonin Reuptakehemmer (SSRIs) entfalten ihre antidepressive Wirkung unter anderem durch die Inhibierung der aktiven Wiederaufnahme des Transmitters in die präsynaptischen Nervenendigungen. Leider tritt dabei die antidepressive Wirkung erst nach einer Behandlung von mindestens 3 Wochen ein, zudem sind ca 30 % der Patienten therapieresistent.

Die Blockade von präsynaptischen Serotonin-Autorezeptoren erhöht durch Aufhebung der negativen Kopplung die Serotoninfreisetzung und damit die aktuelle Transmitterkonzentration im synaptischen Spalt. Dieser Anstieg der Transmitterkonzentration gilt als das antidepressive Wirkprinzip. Dieser Wirkmechanismus unterscheidet sich von den bisher bekannten Antidepressiva, die zugleich die präsynaptischen und somatodendritischen Autorezeptoren aktivieren - und deshalb erst nach Desensibilisierung dieser Autorezeptoren zum verzögerten Wirkungseinstritt führen. Die direkte Autorezeptor-Blockade umgeht diesen Effekt.

Nach bisherigem Wissen handelt es sich bei dem präsynaptischen Serotonin-Autorezeptor um den 5-HT_{1B}-Subtyp (Fink et al., Arch. Pharmacol. 352 (1995), S. 451). Dessen selektive Blockade durch 5-HT_{1B/D}-Antagonisten erhöht die Serotonin-Freisetzung im Gehirn: G.W. Price et al., Behavioural Brain Research 73 (1996), S. 79-82; P.H. Hutson et al., Neuropharmacology Vol. 34, No. 4 (1995), S. 383-392.

Der selektive 5-HT_{1B}-Antagonist GR 127 935 vermindert jedoch überraschenderweise die Serotonin-Freisetzung im Cortex nach systemischer Gabe. Eine Erklärung könnte die Stimulierung von somatodendritischen 5-HT_{1A}-Rezeptoren in der Raphe Region durch das freigesetzte Serotonin sein, die die Feuerrate serotonerger Neuronen und damit die Serotonin-Ausschüttung hemmt (M. Skingle et al., Neuropharmacology Vol. 34 No. 4 (1995), S. 377-382, S. 393-402).

Eine Strategie zur Umgehung der autoinhibitorischen Effekte in serotonergen Ursprungsgebieten verfolgt also die Blockade der präsynaptischen 5-HT_{1B} Rezeptoren. Diese Hypothese wird gestützt durch die Beobachtung, daß der Einfluß von Paroxetine auf die Serotonin-Freisetzung im dorsalen Raphe Nucleus der Ratte durch den 5-HT_{1B}-Rezeptor Antagonisten GR 127 935 potenziert wird (Davidson and Stamford, Neuroscience Letts., 188 (1995),41).

Die zweite Strategie schließt die Blockade beider Typen von Autorezeptoren mit ein, nämlich die 5-HT_{1A}-Rezeptoren, um das neuronale Feuern zu verstärken, und die 5-HT_{1B}-Rezeptoren, um die terminale Serotonin Freisetzung anzuheben (Starkey and Skingle, Neuropharmacology 33 (3-4) (1994),393).

5-HT_{1B/D}-Antagonisten allein oder gekoppelt mit einer 5-HT_{1A}-Rezeptor antagonistischen Komponente sollten deshalb vermehrt die Serotonin-Freisetzung im Gehirn erhöhen und könnten deshalb Vorteile in der Therapie von Depressionen und verwandten psychischen Krankheiten beinhalten.

Es wurde nun gefunden, daß 3-substituierte 3,4,5,6,7,8-Hexahydro-pyrido [4',3':4,5]- thieno [2,3-d] pyrimidin-Derivate der Formel I 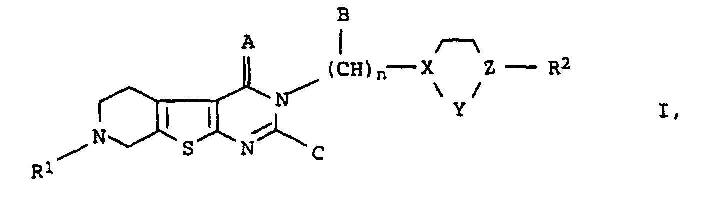 worin
R¹ ein Wasserstoffatom, eine C₁-C₄-Alkylgruppe, eine Acetyl- oder Benzoylgruppe, ein Phenylalkyl C₁-C₄ Rest, wobei der Aromat gegebenenfalls durch Halogen, C₁-C₄-Alkyl-, Trifluormethyl-, Hydroxy-, C₁-C₄-Alkoxy-, Amino-, Cyano- oder Nitrogruppen substituiert ist, ein Naphthylalkyl C₁-C₃-Rest, ein Phenylalkanon C₂-C₃-Rest oder ein Phenylcarbamoylalkyl C₂-Rest bedeutet, wobei die Phenylgruppe durch Halogen substituiert sein kann,
R² eine gegebenenfalls durch Halogenatome, C₁-C₄-Alkyl, Trifluormethyl-, Trifluormethoxy-, Hydroxy-, C₁-C₄-Alkoxy-, Amino-, Monomethylamino-, Dimethylamino-, Cyano- oder Nitrogruppen mono, di- oder trisubstituierte Phenyl-, Pyridyl-, Pyrimidinyl- oder Pyrazinyl-Gruppe darstellt, die gegebenenfalls mit einem Benzolkern,. der gegebenenfalls durch Halogenatome, C₁-C₄-Alkyl, Hydroxy-, Trifluormethyl, C₁-C₄-Alkoxy-, Amino-, Cyano- oder Nitrogruppen mono- oder disubstituiert sein kann und gegebenenfalls 1 Stickstoffatom enthalten kann, oder mit einem 5- oder 6-gliedrigen Ring, der 1-2 Sauerstoffatome enthalten kann, anelliert sein kann,
oder durch eine Phenyl-C₁-C₂-alkyl-bzw.-alkoxy-Gruppe substituiert sein kann, wobei der Phenylrest durch Halogen, eine Methyl-, Trifluormethyl- oder Methoxygruppe substituiert sein kann,
- A: NH oder ein Sauerstoffatom darstellt,
- B: Wasserstoff oder Methyl bedeutet,
- C: Wasserstoff, Methyl oder Hydroxy darstellt,
- X: ein Stickstoffatom bedeutet,
- Y: CH₂, CH₂-CH₂, CH₂-CH₂-CH₂ oder CH₂-CH ist,
- Z: ein Stickstoffatom, Kohlenstoffatom oder CH darstellt, wobei die Bindung zwischen Y und Z auch eine Doppelbindung sein kann,
und n die Zahl 2, 3 oder 4 bedeutet,
und deren Salze mit physiologisch verträglichen Säuren wertvolle pharmakologische Eigenschaften besitzen.

Bevorzugt sind insbesonders Verbindungen, in denen
- R¹: Methyl, Ethyl, Isopropyl, Benzyl, subst. Benzyl, Phenethyl, subst. Phenethyl,
- R²: o-Methoxyphenyl, 1-Naphthyl, Pyrimidin-2-yl, 2-Methoxy-1-naphthyl, 2-Methyl-1-naphthyl,
- A: NH oder ein Sauerstoffatom,
- X: ein Stickstoffatom,
- Y: CH₂-CH₂, CH₂-CH,
- Z: ein Stickstoffatom, Kohlenstoffatom oder CH
und n die Zahl 2 und 3 bedeuten.

Die erfindungsgemäßen Verbindungen der Formel I lassen sich herstellen, indem man eine Verbindung der Formel II 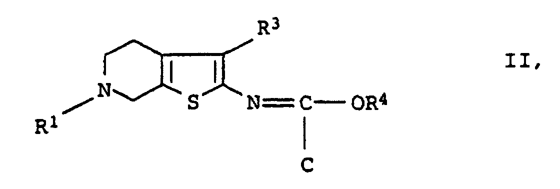 in der R₁ die oben angegebene Bedeutung hat, R³ eine Cyanogruppe oder eine C₁₋₃-Alkyl-carbonsäureestergruppierung darstellt, R⁴ C₁₋₃-Alkyl bedeutet und C Wasserstoff, Methyl oder Hydroxy darstellt, mit einem primären Amin der Formel III 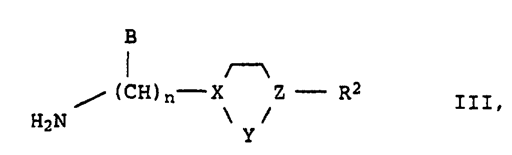 worin R² und B die oben angegebene Bedeutung hat, umsetzt und die so erhaltene Verbindung gegebenenfalls in das Säureadditionssalz einer physiologisch verträglichen Säure überführt.

Die Umsetzung erfolgt zweckmäßig in einem inerten organischen Lösungsmittel, insbesondere einem niederen Alkohol, z.B. Methanol oder Ethanol, oder einem cyclischen gesättigten Ether, insbesondere Tetrahydrofuran oder Dioxan oder ohne Lösungsmittel.

Die Umsetzung erfolgt in der Regel bei Temperaturen von 20 bis 190°C, insbesondere von 60 bis 90°C, und ist im allgemeinen innerhalb von 1 bis 10 Stunden beendet.

Oder man setzt eine Verbindung der Formel II 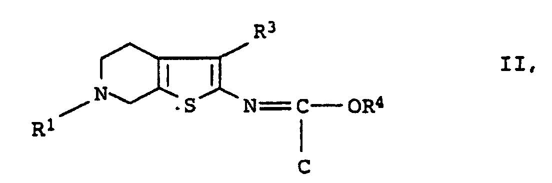 in der R₁ die oben angegebene Bedeutung hat, R³ eine Cyanogruppe oder eine C₁₋₃-Alkyl-carbonsäureestergruppierung darstellt, R⁴ C₁₋₃-Alkyl bedeutet und C Wasserstoff, Methyl oder Hydroxy darstellt, mit einem primären Amin der Formel IV 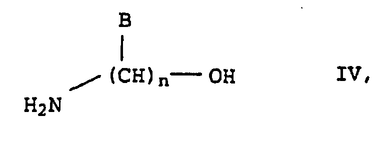 worin B die oben angegebene Bedeutung hat, in einem inerten Lösungsmittel, vorzugsweise Alkoholen wie z.B. Ethanol, bei Temperaturen zwischen 60 und 120°C zum Cyclisierungsprodukt V (D = OH) um 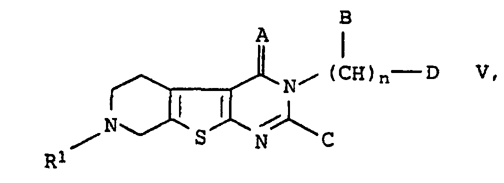 das anschließend mit einem Halogenierungsmittel, wie z.B. Thionylchlorid oder Bromwasserstoffsäure, in einem organischen Lösungsmittel wie einem Halogenkohlenwasserstoff oder ohne Lösungsmittel bei Temperaturen zwischen Raumtemperatur und 100°C in das entsprechende Halogenderivat V (D = Cl, Br) überführt wird. Zuletzt setzt man das Halogenderivat der Formel V (D = Cl, Br) mit einem Amin der allgemeinen Formel VI 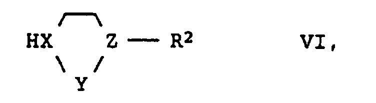 worin X, Y, Z und R² die oben angegebenen Bedeutungen haben, zum erfindungsgemäßen Endprodukt der Formel I um. Diese Umsetzung verläuft am besten in einem inerten organischen Lösungsmittel, vorzugsweise Toluol oder Xylol, in GEgenwart einer Base, wie z.B. Kaliumcarbonat oder Kaliumhydroxyd, bei Temperaturen zwischen 60 und 150°C.

Die erfindungsgemäßen Verbindungen der Formel I können entweder durch Umkristallisation aus den üblichen organischen Lösungsmitteln, bevorzugt aus einem niederen Alkohol, wie Ethanol, umkristallisiert oder durch Säulenchromatographie gereinigt werden.

Die freien 3-substituierten Pyrido [4',3':4,5] thieno [2,3-d]-pyrimidin-Derivate der Formel I können in üblicher Weise in die Säureadditionssalze einer Lösung mit der stöchiometrischen Menge der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure oder Zitronensäure.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 10 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et. al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die als Ausgangsstoffe für die Synthese der neuen Verbindungen benötigten Substanzen der Formel II und III sind bekannt oder lassen sich gemäß den in der Literatur beschriebenen Herstellmethoden aus analogen Ausgangsmaterialien synthetisieren (F. Sauter und P. Stanetty, Monatsh. Chem. (1975), 106(5), 1111-1116; K. Gewald et al, Chem. Ber. 99, 94-100 (1966)).

Die erfindungsgemäßen Verbindungen weisen eine hohe Affinität zu den Serotoninrezeptoren 5-HT_{1B}, 5-HT_{1D} und 5-HT_{1A} auf. Die Affinität zu diesen Rezeptoren ist dabei etwa gleich groß. zumindest in der gleichen Größenordnung. Darüberhinaus weisen einige der erfindungsgemäßen Verbindungen eine gute Serotonin-Reuptake Hemmung auf- ein Prinzip, das bei den meisten Antidepressiva verwirklicht ist.

Diese Verbindungen eignen sich als Arzneimittel zur Behandlung von Krankheitszuständen, bei denen die Serotoninkonzentration erniedrigt ist, und bei denen man im Rahmen einer Therapie gezielt die Aktivität der präsynaptischen Rezeptoren 5-HT_{1B}, 5-HT_{1A}, 5-HT_{1D} blockieren möchte, ohne dabei andere Rezeptoren stark zu beeinflussen. Solch ein Krankheitszustand ist beispielsweise die Depression.

Die Verbindungen der vorliegenden Erfindung können auch für die Behandlung von zentralnervös bedingten Gemütsstörungen wie saisonale affektive Störungen und Dysthymie von Nutzen sein. Dazu gehören auch Angstzustände wie generalisierte Angst, Panikanfälle, Soziophobie, Zwangsneurosen und post-traumatische Stress-Symptome, Gedächtnisstörungen einschließlich Demenz, Amnesien und altersbedingter Gedächtnisschwund sowie psychogene Eßstörungen wie Anorexia nervosa und Bulimia nervosa.

Die erfindungsgemäßen Verbindungen können außerdem für die Behandlung endokriner Erkrankungen wie Hyperprolaktinämie sowie für die Behandlung von Gefäßspasmen (insbesondere der Hirngefäße), Hypertonie und gastrointestinalen Störungen, die mit Motilitäts- und Sekretionsstörungen einhergehen, von Nutzen sein. Ein weiteres Anwendungsgebiet sind Sexualstörungen.

Die folgenden Beispiele dienen zur Erläuterung der Erfindung:

### A Herstellung der Ausgangsmaterialien der Formel II, V und VI

Die als Ausgangsmaterialien eingesetzten 2-Amino-3-carboethoxy(cyano)-4,5,6,7-tetrahydro-thieno[2,3-c]pyridine mit 6-ständiger Methyl-, Benzyl-, Acetyl-, Benzoyl-Gruppe oder mit unsubstituierter 6-Position sind literaturbekannt (K. Gewald et al.).
a) 2-Ethoxymethylen-amino-3-cyano-6-methyl-4,5,6,7-tetrahydrothieno [2,3-c] pyridin
   46,0 g (238 mM) 2-Amino-3-cyano-6-methyl-4,5,6,7-tetrahydrothieno [2,3-c] pyridin in 250 ml Triethylorthoformiat wurden mit 3,5 ml Acetanhydrid versetzt und unter Stickstoff 4 h am Rückfluß gekocht. Danach saugte man den Ansatz über eine Nutsche heiß ab und engte das Filtrat bei 80°C am Rotationsverdampfer ganz ein. Den Rückstand nahm man in 300 ml Methylt-butyl-ether auf und erhitzte zum Sieden. Nach Absaugen der unlöslichen Festkörper kristallisierten im Eisbad unter Rühren 45,4 g (77 %) Produkt aus. Aus der Mutterlauge erhielt man noch 1,7 g (3 %) Produkt als zweite Fraktion.
   Schmp.: 88-89°C.
b) 2-Ethoxymethylen-amino-3-carboethoxy-6-methyl-4,5,6,7-tetrahydro-thieno [2,3-c] pyridin
   40,0 g (167 mM) 2-Amino-3-carboethoxy-6-methyl-4,5,6,7-tetrahydro-thieno [2,3-c] pyridin in 250 ml Triethylorthoformiat wurden mit 3,2 ml Acetanhydrid versetzt und unter Stickstoff 3 h am Rückfluß gekocht. Danach engte man den Ansatz bei 80°C am Rotationsverdampfer ganz ein. Man isolierte 48,0 g (97 %) Rohprodukt als dunkles Öl, das für die weitere Umsetzung genügend rein ist.
c) 2-Amino-3-carboethoxy-6-(4-chlor)-benzyl-4,5,6,7-tetrahydrothieno [2,3-c] pyridin
   20,4 g (90,2 mM)2-Amino-3-carboethoxy -4,5,6,7-tetrahydrothieno [2,3-c] pyridin in 250 ml Tetrahydrofuran wurden mit 25,6 g (204 mM) 4-Chlor-benzylchlorid und 12,4 g (90 mM) fein pulverisiertem Kaliumcarbonat versetzt und 3 h am Rückfluß gekocht. Danach engte man den Ansatz am Rotationsverdampfer ganz ein. Den Rückstand verteilte man zwischen Methyl-tbutyl-ether und Wasser, stellte mit Natronlauge alkalisch, wusch die organische Phase mit Wasser und engte ein. Das Rohprodukt löste man in 100 ml heißen Ethanol und ließ unter Rühren kristallisieren. Man isolierte 20,5 g (65 %) Produkt mit Schmp. 134-135°C.
d) 2-Ethoxymethylen-amino-3-carboethoxy-6-(4-chlor)-benzyl-4,5,6,7-tetrahydro-thieno[2,3-c]pyridin
   19,3 g (55,0 mM) 2-Amino-3-carboethoxy-6-(p-chlorbenzyl)-4,5,6,7-tetrahydro-thieno [2,3-c] pyridin in 125 ml Triethylorthoformiat wurden mit 2,0 ml Acetanhydrid versetzt und unter Stickstoff 1 h am Rückfluß gekocht. Danach engte man den Ansatz bei 80°C am Rotationsverdampfer ganz ein. Man isolierte 21,9 g (98 %) Rohprodukt als dunkles Öl, das für die weitere Umsetzung genügend rein ist.
e) 2-Amino-3-carboethoxy-6-(3-phenyl)-propyl-4,5,6,7-tetrahydrothieno[2,3-c]pyridin
   10,0 g (44,2 mM) 2-Amino-3-carboethoxy-4,5,6,7-tetrahydrothieno[2,3-c)pyridin in 100 ml Xylol wurden mit 9,0 g (45 mM) 1-Phenyl-3-brom-propan, 400 mg Kaliumjodid und 6,1 g (44,2 mM) fein pulverisiertem Kaliumcarbonat versetzt und 6 h am Rückfluß gekocht. Nach Einengen am Rotationsverdampfer nahm man den Rückstand in Wasser auf, stellte auf pH = 10 und extrahierte zweimal mit Methylenchlorid. Nach Trocknen und Einengen der organischen Phase rührte man das Rohprodukt in 50 ml Isopropanol aus. Die hellen Festkörper wurden abgesaugt und mit Isopropanol nachgewaschen. Man isolierte 7,8 g (51 %) Produkt mit Schmp. 108-110°C.
   Analog c) und e) stellte man die weiteren in 6-Position substituierten 4,5,6,7-Tetrahydro-thieno[2,3-c]pyridin Derivate her, z.B.:
   2-Amino-3-carboethoxy-6-ethyl-4,5,6,7-tetrahydro-thieno[2,3-c]pyridin, Schmp. 74-76°C
   2-Amino-3-carboethoxy-6-isopropyl-4,5,6,7-tetrahydro-thieno-[2,3-c]pyridin
   2-Amino-3-carboethoxy-6-benzyl-4,5,6,7-tetrahydro-thieno-[2,3-c]pyridin, Schmp. 116-118°C
   2-Amino-3-carboethoxy-6-(4-methyl)-benzyl-4,5,6,7-tetrahydrothieno[2,3-c]pyridin
   2-Amino-3-carboethoxy-6-(4-nitro)-benzyl-4,5,6,7-tetrahydrothieno[2,3-c]pyridin, Schmp. 170-172°C
   2-Amino-3-carboethoxy-6-(4-methoxy)-benzyl-4,5,6,7-tetrahydro-thieno[2,3-c]pyridin, Schmp. 154-156°C
   2-Amino-3-carboethoxy-6-(2-phenyl)-ethyl-4,5,6,7-tetrahydrothieno[2,3-c]pyridin, Schmp. 80-83°C
   2-Amino-3-carboethoxy-6-(2-(4-methoxy-phenyl)-ethyl)-4,5,6,7-tetrahydro-thieno[2,3-c]pyridin, Schmp. 76-78°C
   2-Amino-3-carboethoxy-6-(2-(4-chlor-phenyl)-ethyl)-4,5,6,7-tetrahydro-thieno[2,3-c]pyridin, Schmp. 102-105°C
   2-Amino-3-carboethoxy-6-(3-(4-chlor)-phenyl)-propyl-4,5,6,7-tetrahydro-thieno[2,3-c]pyridin
   2-Amino-3-carboethoxy-6-(4-pheny)-butyl-4,5,6,7-tetrahydrothieno [2,3-c]pyridin
   2-Amino-3-carboethoxy-6-(3-benzoyl)-propyl-4,5,6,7-tetrahydro-thieno[2,3-c]pyridin
   2-Amino-3-carboethoxy-6-(2-benzoyl-amino)-ethyl-4,5,6,7-tetrahydro-thieno[2,3-c]pyridin, Schmp. 190-192°C
   2-Amino-3-carboethoxy-6-(3-benzoyl-amino)-propyl-4,5,6,7-tetrahydro-thieno[2,3-c]pyridin
f) N-(3-Carboethoxy-6-methyl-4,5,6,7-tetrahydro-thieno-[2,3-c]pyridin-2-yl)-ethanimidsäureethylester
   3,0 g (12,5 mM) 2-Amino-3-carboethoxy-4,5,6,7-tetrahydrothieno[2,3-c]pyridin in 25 ml Triethylorthoacetat wurden mit 0,8 ml Acetanhydrid versetzt und unter Stickstoff 2 h am Rückfluß gekocht. Danach engte man den Ansatz bei 80°C am Rotationsverdampfer ganz ein. Man isolierte 3,6 g (93 %) Rohprodukt als dunkles Öl, das für die weitere Umsetzung genügend rein ist.
g) 2-Carboethoxy-amino-3-carboethoxy-6-acetyl-4,5,6,7-tetrahydro-thieno[2,3-c]pyridin
   5,0 g (18,6 mM) 2-Amino-3-carboethoxy-6-acetyl-4,5,6,7-tetrahydro-thieno[2,3-c]pyridin in 50 ml Toluol wurden mit 3,0 g (28 mM) Chlorameisensäureethylester und 2,6 g (18,6 mM) fein pulverisiertem Kaliumcarbonat versetzt und 2 h am Rückfluß gekocht. Danach nahm man die Reaktionsmischung in Eis/Wasser auf, trennte die Toluolphase ab und extrahierte die wäßrige Phase mit Toluol nach. Die vereinigten organischen Phasen wurden nach dem Trocknen eingeengt. Man isolierte 5,8 g (92 %) Produkt als Öl, das langsam etwas durchkristallisiert.
h) 3,4,5,6,7,8-Hexahydro-3-(2-hydroxy)-ethyl-7-methyl-pyrido-[4',3':4,5]thieno[2,3-d]pyrimidin-4-on
   86,4 g (292 mM) 2-Ethoxymethylen-amino-3-carboethoxy-6-methyl-4,5,6,7-tetrahydro-thieno[2,3-c]pyridin in 200 ml Ethanol wurden mit 17,6 ml (292 mM) Ethanolamin versetzt und 2 h am Rückfluß gekocht. Anschließend engte man den Ansatz im vakuum ein und nahm den Rückstand unter Rühren in 30 ml Essigester auf. Die über Nacht ausgefallenen Festkörper saugte man ab und wusch mit wenig Essigester nach. Nach Umkristallisieren aus Ethanol isolierte man 48,0 g (62 %) Produkt mit Schmp. 163-165°C.
i) 3,4,5,6,7,8-Hexahydro-3-(2-chlor)-ethyl-7-methyl-pyrido-[4',3':4,5]thieno[2,3-d]pyrimidin-4-on
   42,0 g (158 mM) 3,4,5,6,7,8-Hexahydro-3-(2-hydroxy)-ethyl-7-methyl-pyrido[4',3':4,5]thieno[2,3-d]pyrimidin-4-on in 240 ml 1,2-Dichlorethan wurden auf Rückfluß erhitzt und anschließend 12,7 ml (175 mM) Thionylchlorid in 20 ml 1,2-Dichlorethan zugetropft. Nach 2 h Rückflußkochen ließ man das Reaktionsgemisch abkühlen und goß auf Eis/Wasser. Man verteilte bei pH = 10 zwischen Methylenchlorid und Wasser und extrahierte die wäßrige Phase mit Methylenchlorid nach. Nach Trocknen engte man die vereinigten organischen Phasen ein. Das Rohprodukt (40 g) kristallisierte man aus 400 ml Isopropanol um. Man isolierte 30,5 g (68 %) Produkt mit Schmp. 159-161°C.
   Analog h) und i) stellte man her:
   3,4,5,6,7,8-Hexahydro-3-(1-hydroxy)-prop-2-yl-7-methylpyrido[4',3':4,5]thieno[2,3-d]pyrimidin-4-on
   3,4,5,6,7,8-Hexahydro-3-(1-chlor)-prop-2-yl-7-methyl-pyrido-[4',3':4,5]thieno[2,3-d]pyrimidin-4-on
   3,4,5,6,7,8-Hexahydro-3-(2-hydroxy)-propyl-7-methyl-pyrido-[4',3':4,5]thieno[2,3-d]pyrimidin-4-on, Schmp. 158-160°C
   3,4,5,6,7,8-Hexahydro-3-(2-chlor)-propyl-7-methyl-pyrido-[4',3':4,5]thieno[2,3-d]pyrimidin-4-on
k) N-(1-Naphthyl)-piperazin
   Zu einer Mischung aus 5.4 g (24.2 mM) Palladiumacetat und 14,7 g (48.3 mM) Tri-o-tolylphosphin in 500 ml Xylol wurden 83,2 g (966 mM) Piperazin, 38.0 g (339 mM) Kalium-tert.butylat und 50.0 g (241 mM) 1-Bromnaphthalin zugegeben und die Reaktionsmischung 10 h unter gutem Rühren und unter Stickstoffatmosphäre auf Rückfluß erhitzt. Danach verdünnte man den Ansatz mit Methylenchlorid, filtrierte die unlöslichen Rückstände ab und engte das Filtrat ein. Das Rohprodukt reinigte man durch Säulenchromatographie (Kieselgel, Laufmittel THF/Methanol/Ammoniak 85/13/2). Man isolierte 21.5 g (42 %) Produkt mit Schmp. 84-86°C.
l) N-(2-Methyl-1-naphthyl)-piperazin
   13.0 g (82.7 mM) l-Amino-2-methyl-naphthalin in 100 ml Chlorbenzol wurden mit 14,7 g (82.7 mM) Bis-(2-chlorethyl)-amin x HCl versetzt und 90 h unter Stickstoff bei Rückfluß gekocht. Anschließend engte man den Ansatz ein, verteilte zwischen Methylenchlorid und Wasser bei pH=9 und engte die organische Phase nach Trocknen ein. Das Rohprodukt reinigte man durch Säulenchromatographie (Kieselgel, Laufmittel /THF/Methanol/Ammoniak 85/13/2. Man isolierte 11.6 g (62 %) Produkt.
m) 4-Piperazin-1-yl-isochinolin
   Es wurden 4.51 g (21.7 mM) 4-Bromisochinolin, 4.65 g (25.0 mM) Piperazin-N-carbonsäure-t-butylester, 0.1 g (0.11 mM) Tris-(dibenzylidenaceton)-dipalladium, 0.11 g (0.18 mM) 2,2'-Bis-(diphenylphosphino)-1,1'-binaphthyl und 2.92 g (30.4 mM) Natrium-t-butylat in 50 ml Toluol zusammengegeben und 2 h bei 75°C gerührt. Man gab die Reaktionsmischung auf Eis/Kochsalz, extrahierte mit Essigester, trocknete die organische Phase über Natriumsulfat und entfernte das Lösungsmittel am Rotationsverdampfer. Das Produkt kristallisierte aus, wurde abgesaugt und mit Pentan gewaschen. Man erhielt 5.5 g (81 %) des Boc-geschützten Piperazins (Schmp.: 111°C). Es wurden 5.2 g (16.6 mM) dieser Substanz in 17 ml Dichlormethan aufgenommen und bei 0°C langsam mit 17 ml Dichlormethan aufgenommen und bei 0°C langsam mit 17 ml (0.22 mM) Trifluoressigsäure versetzt. Man ließ 4 h bei 0°C rühren, goß auf Eiswasser und extrahierte mit Dichlormethan. Die wässrige Phase wurde filtriert, alkalisch eingestellt und mit Dichlormethan extrahiert. Nach dem Trocknen über Natriumsulfat und dem weitgehenden Entfernen des Lösungsmittels verdünnte man mit Diethylether und fällte das Hydrochlorid mit etherischer Salzsäure. Man erhielt 3.2 g (67 %) des Produktes mit Schmp. 293-294°C.
   Analog k), l) und m) stellte man weitere Piperazinderivate (siehe Beispiele) her, soweit sie nicht literaturbekannt waren (vgl. auch Patentanmeldung DE 19636769.7).

### B Herstellung der Endprodukte

### Beispiel 1

### 3,4,5,6,7,8-Hexahydro-7-methyl-3-[2-(4-(2-methoxy-phenyl)-piperazin-1-yl)-ethyl]-pyrido[4',3':4,5]thieno[2,3-d] pyrimidin-4-imin x 3 HCl

3,0 g (12,1 mM) 2-Ethoxymethylen-amino-3-cyano-6-methyl-4,5,6,7-tetrahydro-thieno [2,3-c] pyridin in 60 ml Ethanol wurden mit 3,3 g (12,1 mM) 1-(2-Amino-ethyl)-4-(2-methoxy-phenyl)-piperazin versetzt und 3 h am Rückfluß gekocht. Danach engte man den Ansatz am Rotationsverdampfer ein und nahm den Rückstand in 100 ml Essigester auf. Man fällte unter Rühren durch Zugabe von etherischer Salzsäure das Trihydrochlorid, saugte das Produkt unter Stickstoff ab und wusch mit Essigester nach. Nach Trocknen bei 50°C im Vakuumschrank isolierte man 3,6 g (55 %) Produkt mit dem Zersp. 282-284°C.

### Beispiel 2

### 3,4,5,6,7,8-Hexahydro-7-methyl-3-[2-(4-(2-methoxy-phenyl)-piperazin-1-yl)-ethyl]-pyrido[4',3':4,5]thieno[2,3-d]pyrimidin-4-on x 3 HCl

3,0 g (12,1 mM) 2-Ethoxymethylen-amino-3-carboethoxy-6-methyl-4,5,6,7-tetrahydro-thieno [2,3-c] pyridin in 50 ml Ethanol wurden mit 2,4 g (10,2 mM) 1-(2-Amino-ethyl)-4-(2-methoxy-phenyl)-piperazin versetzt und 3 h am Rückfluß gekocht. Danach engte man den Ansatz am Rotationsverdampfer ein und reinigte das Rohprodukt durch Säulenchromatographie (Kieselgel, Laufmittel Methylenchlorid/Methanol 93/7). Man überführte die freie Base wie oben in das Trihydrochlorid (3,2 g, 48 %) mit Zersp. 288-290°C.

### Beispiel 3

### 3,4,5,6,7,8-Hexahydro-7-(4-chlorbenzyl)-3-[2-(4-(2-methoxyphenyl)-piperazin-1-yl)-ethyl]-pyrido[4',3':4,5]thieno[2,3-d] pyrimidin-4-on x 3 HCl

3,5 g (8,6 mM) 2-Ethoxymethylen-amino-3-carboethoxy-6-(4-chlorbenzyl)-4,5,6,7-tetrahydro-thieno [2,3-c] pyridin in 60 ml Ethanol wurden mit 2,0 g (8,6 mM) 1-(2-Amino-ethyl)-4-(2-methoxyphenyl)-piperazin versetzt und 4 h am Rückfluß gekocht. Danach engte man den Ansatz am Rotationsverdampfer ein und reinigte das Rohprodukt durch Säulenchromatographie (Kieselgel, Laufmittel Methylenchlorid/Methanol 95/5). Man überführte die freie Base wie oben in das Trihydrochlorid (3,2 g, 57 %) mit Zersp. 290-293°C.

### Beispiel 4

### 3,4,5,6,7,8-Hexahydro-7-methyl-3-[3-(4-(2-methoxy-phenyl)-piperazin-1-yl)-propyl)-pyrido[4',3':4,5]thieno[2,3-d]pyrimidin-4-on x 3 HCl x 2 H₂O

3,5 g (11,8 mM) 2-Ethoxymethylen-amino-3-carboethoxy-6-methyl-4,5,6,7-tetrahydro-thieno [2,3-c] pyridin in 40 ml Ethanol wurden mit 3,0 g (11,8 mM) 1-(3-Amino-propyl)-4-(2-methoxy-phenyl)-piperazin versetzt und 2 h am Rückfluß gekocht. Danach engte man den Ansatz am Rotationsverdampfer ein und reinigte das Rohprodukt durch Säulenchromatographie (Kieselgel, Laufmittel Methylenchlorid/Methanol 93/7). Man überführte die freie Base wie oben in das Trihydrochlorid (3,1 g, 44 %) mit Zersp. 122-124°C.

### Beispiel 5

### 3,4,5,6,7,8-Hexahydro-7-methyl-3-[3-(4-pyridin-2-yl)-piperazin-1-yl)-propyl]-pyrido[4',3':4,5]thieno[2,3-d]pyrimidin-4-imin x 4 HCl x H₂O

3,0 g (12,1 mM) 2-Ethoxymethylen-amino-3-cyano-6-methyl-4,5,6,7-tetrahydro-thieno [2,3-c] pyridin in 60 ml Ethanol wurden mit 2,65 g (12,1 mM) 1-(3-Amino-propyl)-4-pyridin-2-yl-piperazin versetzt und 6 h am Rückfluß gekocht. Danach engte man den Ansatz am Rotationsverdampfer ein und nahm das Rohprodukt in 100 ml Essigester auf. Die über Nacht kristallisierten Festkörper überführte man wie oben in das Tetrahydrochlorid. Man isolierte 2,7 g (38 %) Produkt mit Zersp. 261-264°C.

### Beispiel 6

### 3,4,5,6,7,8-Hexahydro-7-methyl-3-[3-(4-(2-thiomethyl-phenyl)-piperazin-1-yl)-propyl]-pyrido[4',3':4,5]thieno[2,3-d] pyrimidin-4-imin x 3 HCl

3,0 g (12,1 mM) 2-Ethoxymethylen-amino-3-cyano-6-methyl-4,5,6,7-tetrahydro-thieno [2,3-c] pyridin in 50 ml Ethanol wurden mit 3,2 g (12,1 mM) 1-(3-Amino-propyl)-4-(2-thiomethyl-phenyl)-piperazin versetzt und 4 h am Rückfluß gekocht. Danach engte man den Ansatz am Rotationsverdampfer ein und nahm den Rückstand in 100 ml Essigester unter Erhitzen zum Sieden auf. Nach dem Abkühlen filtrierte man die unlöslichen Anteile, fällte im Filtrat unter Rühren durch Zugabe von etherischer Salzsäure das Trihydrochlorid, saugte das Produkt unter Stickstoff ab und wusch mit Essigester nach. Das Rohprodukt (5,1 g) wurde anschließend aus Methanol umkristallisiert. Man isolierte 3,8 g (54 %) Produkt mit Schmp. 306-307°C.

### Beispiel 7

### 3,4,5,6,7,8-Hexahydro-7-methyl-3-[2-(4-pyridin-2-yl-piperazin-1-yl)-ethyl]pyrido[4',3':4,5]thieno[2,3-d]pyrimidin-4-on x 3HCl x 2H₂O

2,2 g (7,8 mM) 3,4,5,6,7,8-Hexahydro-3-(2-chlor)-ethyl-7-methylpyrido[4',3':4,5]thieno[2,3-d]pyridimin-4-on in 50 ml Xylol wurden mit 1,6 g (10,0 mM) 1-(2-Pyridyl)-piperazin, 1,4 g (10,0 mM) fein pulverisiertem Kaliumcarbonat sowie 400 mg Kaliumjodid versetzt und 24 h am Rückfluß gekocht. Danach engte man den Ansatz am Rotationsverdampfer ein und verteilte den Rückstand zwischen Methylenchlorid und Wasser bei pH = 10. Die wäßrige Phase extrahierte man nocheinmal mit Methylenchlorid nach und engte die vereinigten organischen Phasen nach dem Trocknen ein. Das Rohprodukt reinigte man durch Säulenchromatographie (Kieselgel, Laufmittel Aceton). Man isolierte 2,3 g (72 %) Produkt, das in 100 ml Essigester gelöst und mit HCl/Essigester-Lösung in das Hydrochlorid mit Schmp. 233-235°C übergeführt wurde.

### Beispiel 8

### 3,4,5,6,7,8-Hexahydro-7-methyl-3-[1-(4-(1-naphthyl)-piperazin-1-yl)-prop-2-yl]-pyrido[4',3':4,5]thieno[2,3-d]pyrimidin-4-on x 3HCl x 2H₂O

2,7 g (9,0 mM) 3,4,5,6,7,8-Hexahydro-3-(1-chlor)-prop-2-yl-7-methyl-pyrido[4',3':4,5]thieno[2,3-d]pyrimidin-4-on in 50 ml Xylol wurden mit 2,1 g (10,0 mM) 1-(1-Naphthyl)-piperazin, 1,4 g (10,0 mM) fein pulverisiertem Kaliumcarbonat sowie 250 mg Kaliumjodid versetzt und 70 h am Rückfluß gekocht. Danach engte man den Ansatz am Rotationsverdampfer ein und verteilte den Rückstand zwischen Methylenchlorid und Wasser bei pH = 10. Die wäßrige Phase extrahierte man nocheinmal mit Methylenchlorid nach und engte die vereinigten organischen Phasen nach dem Trocknen ein. Das Rohprodukt reinigte man durch Säulenchromatographie (Kieselgel, Laufmittel Aceton). Man isolierte 1,6 g (38 %) Produkt, das in Essigester gelöst und mit HCl/Essigester-Lösung in das Hydrochlorid mit Schmp. 242-244°C übergeführt wurde.

### Beispiel 9

### 3,4,5,6,7,8-Hexahydro-7-methyl-3-[2-(4-(2-methoxyphenyl)-piperazin-1-yl)-propyl]-pyrido[4',3':4,5]thieno[2,3-d]pyrimidin-4-on x 3HCl

2,9 g (8,9 mM) 3,4,5,6,7,8-Hexahydro-3-(2-chlor)-propyl-7-methylpyrido[4',3':4,5]thieno[2,3-d]pyrimidin-4-on in 60 ml Xylol wurden mit 3,5 g (18,0 mM) 1-(2-Methoxyphenyl)-piperazin, 1,4 g (10,0 mM) fein pulverisiertem Kaliumcarbonat sowie 400 mg Kaliumjodid versetzt und 100 h am Rückfluß gekocht. Danach engte man den Ansatz am Rotationsverdampfer ein und verteilte den Rückstand zwischen Methylenchlorid und Wasser bei pH = 10. Die wäßrige Phase extrahierte man nocheinmal mit Methylenchlorid nach und engte die vereinigten organischen Phasen nach dem Trocknen ein. Das Rohprodukt reinigte man durch Säulenchromatographie (Kieselgel, Laufmittel Aceton). Man isolierte 1,0 g (25 %) Produkt, das in 100 ml Essigester gelöst und mit HCl/Essigester-Lösung in das Hydrochlorid mit Schmp. 190-192°C (Zers.) übergeführt wurde.

### Beispiel 10

### 3,4,5,6,7,8-Hexahydro-2,7-dimethyl-3-[2-(4-(2-methoxy-phenyl)-piperazin-1-yl)-ethyl]-pyrido[4',3':4,5]thieno[2,3-d]pyrimidin-4-on

1,9 g (6,2 mM) N-(3-Carboethoxy-6-methyl-4,5,6,7-tetrahydrothieno[2,3-c]pyridin-2-yl)-ethanimidsäureethylester in 30 ml Ethanol wurden mit 1,5 g (6,2 mM) 1-(2-Amino-ethyl)-4-(2-methoxyphenyl)-piperazin versetzt und 7 h am Rückfluß gekocht. Danach engte man den Ansatz am Rotationsverdampfer ein und nahm den Rückstand in 20 ml Essigester auf. Über Nacht kristallisierten 2,1 g Rohprodukt aus, das abgesaugt und durch Säulenchromatographie (Kieselgel, Laufmittel Methylenchlorid/Methanol 92/8). Man isolierte 0,8 g (29 %) Produkt.

### Beispiel 11

### 3,4,5,6,7,8-Hexahydro-2-hydroxy-7-acetyl-3-[2-(4-(2-methoxyphenyl)-piperazin-1-yl)-ethyl]-pyrido[4',3'-4,5]thieno[2,3-d]-pyrimidin-4-on

2,5 g (7,3 mM) 2-Carboethoxy-amino-3-carboethoxy-6-acetyl-4,5,6,7-tetrahydro-thieno[2,3-c]pyridin wurden mit 1,7 g (7,3 mM) 1-(2-Amino-ethyl)-4-(2-methoxy-phenyl)-piperazin 2 h auf 180°C unter Stickstoff und gutem Rühren der Schmelze erhitzt. Nach dem Abkühlen reinigte man das Rohprodukt durch Säulenchromatographie (Kieselgel, Laufmittel Methylenchlorid/Methanol 95/5). Man isolierte 0,7 g (20 %) Produkt mit Schmp. 135-137°C.

### Beispiel 12

### 3,4,5,6,7,8-Hexahydro-7-acetyl-3-[2-(4-(2-methoxy-phenyl)-piperazin-1-yl)-ethyl]pyrido[4',3':4,5]thieno[2,3-d]pyrimidin-4-on

5,8 g (23,4 mM) 2-Ethoxymethylen-amino-3-carboethoxy-6-acetyl-4,5,6,7-tetrahydro-thieno[2,3-c]pyridin in 50 ml Ethanol wurden mit 5,5 g (23,4 mM) 1-(2-Aminoethyl)-4-(2-methoxy-phenyl)-piperazin versetzt und 2 h am Rückfluß gekocht. Danach engte man den Ansatz am Rotationsverdampfer ein und nahm den Rückstand in 30 ml Essigester auf, erhitzte zum Sieden und ließ unter Rühren abkühlen. Die auskristallisierten Festkörper wurden nach Kühlen im Eisbad abgesaugt und mit Essigester nachgewaschen. Man isolierte 8,7 g (80 %) Produkt mit Schmp. 170-172°C.

### Beispiel 13

### 3,4,5,6,7,8-Hexahydro-3-[2-(4-(2-methoxy-phenyl)-piperazin-1-yl)-ethyl]-pyrido[4',3':4,5]thieno[2,3-d]pyrimidin-4-on

4,0 g (8.6 mM) 3,4,5,6,7,8-Hexahydro-7-acetyl-3-[2-(4-(2-methoxyphenyl)-piperazin-1-yl)-ethyl]-pyrido[4',3':4,5]thieno[2,3-d]-pyrimidin-4-on wurden in 80 ml 10%iger Salzsäure gelöst und 2 h bei 100°C Badtemperatur gerührt. Danach goß man den Ansatz auf Eiswasser, stellte mit konz. Natronlauge alkalisch und extrahierte zweimal mit Methylenchlorid. Die vereinigten organischen Phasen wurden getrocknet und eingeengt. Man isolierte 3,7 g Rohprodukt, das aus 50 ml Isopropanol umkristallisiert wurde. Man erhielt 2,4 g (66 %) Produkt mit Schmp. 168-170°C.

### Beispiel 14

### 3,4,5,6,7,8-Hexahydro-7-(2-(1-naphthyl)-ethyl)-3-[2-(4-(2-methoxy-phenyl)-piperazin-1-yl)-ethyl]-pyrido[4',3':4,5]thieno-[2,3-d]pyrimidin-4-on x 3HCl

1,0 g (2,3 mM) 3,4,5,6,7,8-Hexahydro-3-[2-(4-(2-methoxy-phenyl)-piperazin-1-yl)-ethyl]-pyrido[4',3':4,5]thieno[2,3-d]pyrimidin-4-on in 35 ml Xylol wurden mit 0,8 g (3,4 mM) 2-Brom-1-naphth-1-yl-ethan sowie mit 0,3 g (2,4 mM) fein pulverisiertem Kaliumcarbonat versetzt und 12 h am Rückfluß gekocht. Danach engte man den Ansatz am Rotationsverdampfer ein und verteilte den Rückstand bei pH =10 zwischen Methylenchlorid und Wasser. Die wäßrige Phase wurde nochmals mit Methylenchlorid nachextrahiert. Die vereinigten organischen Phasen engte man nach dem Trocknen ein. Man erhielt 2,7 g Rohprodukt als dunkles Öl, das durch Säulenchromatographie (Kieselgel. Laufmittel Methylenchlorid/Aceton 7/3). Man isolierte nach Überführung in das Hydrochlorid in Essigester 1,0 g (63 %) Produkt mit Schmp. 293-295°C (Zers.).

Analog der Beispiele 1 bis 14 wurden hergestellt:
15. 3,4,5,6,7,8-Hexahydro-7-methyl-3-[3-(4-(2-methoxy-phenyl)-piperazin-1-yl)-propyl]-pyrido[4',3':4,5]thieno[2,3-d]pyrimid in-4-imin, Schmp. 112-114°C
16. 3,4,5,6,7,8-Hexahydro-7-benzyl-3-[3-(4-(2-methoxy-phenyl)-piperazin-1-yl)-propyl]-pyrido[4',3':4,5]thieno[2,3-d]pyrimid in-4-imin x 2 HCl, Schmp. 258-261°C (Zers.)
17. 3,4,5,6,7,8-Hexahydro-7-benzyl-3-[2-(4-phenyl-piperazin-1-yl)-ethyl]-pyrido[4',3':4,5]thieno[2,3-d]pyrimidin-4-imin, Schmp. 168-170°C
18. 3,4,5,6,7,8-Hexahydro-7-benzyl-3-[3-(4-(2-methoxy-phenyl)-piperazin-1-yl)-propyl]-pyrido[4',3':4,5]thieno[2,3-d] pyrimidin-4-on, Schmp. 66-67°C
19. 3,4,5,6,7,8-Hexahydro-7-benzyl-3-[2-(4-phenyl-piperazin-1-yl)-ethyl]-pyrido[4',3':4,5]thieno[2,3-d]pyrimidin-4-on, Schmp. 70-71°C
20. 3,4,5,6,7,8-Hexahydro-7-methyl-3-[2-(4-pyrimidin-2-ylpiperazin-1-yl)-ethyl]-pyrido[4',3':4,5]thieno[2,3-d]-pyrimidin-4-imin Tritartrat, Schmp. 112-114°C (Zers.)
21. 3,4,5,6,7,8-Hexahydro-7-methyl-3-[3-(4-(3-methoxy-phenyl)-piperazin-1-yl]-propyl]-pyrido[4',3':4,5]thieno[2,3-d]-pyrimidin-4-imin x 3 HCl x 2 H₂O, Schmp. 268-270°C (Zers.)
22. 3,4,5,6,7,8-Hexahydro-7-methyl-3-[3-(4-napht-1-yl-piperazin-1-yl)-propyl]-pyrido[4',3':4,5]thieno[2,3-d]pyrimidin-4-imin x 3 HCl, Schmp. 250-253°C (Zers.)
23. 3,4,5,6,7,8-Hexahydro-7-methyl-3-[3-(4-(2-nitro-phenyl)-piperazin-1-yl)-propyl]-pyrido[4',3':4,5]thieno[2,3-d]-pyrimidin-4-imin x 3 HCl x 2 H₂O, Schmp. 271-273°C (Zers.)
24. 3,4,5,6,7,8-Hexahydro-7-methyl-3-[3-(4-(2-methyl)-piperazin-1-yl)-propyl]-pyrido[4',3':4,5]thieno[2,3-d]pyrimidin-4-imin x 3 HCl Schmp. 280-282°C (Zers.)
25. 3,4,5,6,7,8-Hexahydro-7-methyl-3-[3-(4-(2-amino-phenyl)-piperazin-1-yl]-propyl]-pyrido[4',3':4,5]thieno[2,3-d]-pyrimidin-4-imin x HCl x 4 H₂O, Schmp. 113-115°C (Zers.)
26. 3,4,5,6,7,8-Hexahydro-7-methyl-3-[3-(4-(2-chlor-phenyl)-piperazin-1-yl)-propyl]-pyrido[4',3':4,5]thieno[2,3-d]-pyrimidin-4-imin x 3 HCl, Schmp. 261-263°C (Zers.)
27. 3,4,5,6,7,8-Hexahydro-7-methyl-3-[2-(4-pyrimidin-2-ylpiperazin-1-yl)-ethyl]-pyrido[4',3':4,5]thieno[2,3-d]-pyrimidin-4-on, Schmp. 146-148°C
28. 3,4,5,6,7,8-Hexahydro-7-methyl-3-[3-(4-benzyl-piperidin-1-yl)-propyl]-pyrido[4',3':4,5]thieno[2,3-d]pyrimidin-4-imin x 3 HCl, Schmp. 295-297°C (Zers.)
29. 3,4,5,6,7,8-Hexahydro-7-methyl-3-[3-(4-(2-hydroxy-phenyl)-piperazin-1-yl)-propyl]-pyrido[4',3':4,5]thieno[2,3-d]-pyrimidin-4-imin, Schmp. 164-166°C
30. 3,4,5,6,7,8-Hexahydro-7-methyl-3-[4-(4-(2-methoxy-phenyl)-piperazin-1-yl)-butyl]-pyrido[4',3';4,5]thieno[2,3-d]-pyrimidin-4-imin x HCl x 3 H₂O, Schmp. 272-274°C (Zers.)
31. 3,4,5,6,7,8-Hexahydro-7-methyl-3-[3-(4-(2-ethoxy-phenyl)-piperazin-1-yl)-propyl]-pyrido[4',3':4,5]thieno[2,3-d]-pyrimidin-4-imin x 3 HCl x 3 H₂O, Schmp. 284-286°C (Zers.)
32. 3,4,5,6,7,8-Hexahydro-7-methyl-3-[3-(4-(2-ethyl-phenyl)-piperazin-1-yl)-propyl]-pyrido[4',3':4,5]thieno[2,3-d]-pyrimidin-4-imin x 3 HCl, Schmp. 303-305°C (Zers.)
33. 3,4,5,6,7,8-Hexahydro-7-methyl-3-[3-(4-(2-cyano-phenyl)-piperazin-l-yl)-propyl]-pyrido[4',3':4,5]thieno[2,3-d]-pyrimidin-4-on x 2 HCl x H₂O, Schmp. 136-138°C (Zers.)
34. 3,4,5,6,7,8-Hexahydro-7-methyl-3-[3-(4-phenyl-piperidin-1-yl)-propyl]-pyrido[4',3':4,5]thieno[2,3-d]pyrimidin-4-imin x 3 HCl, Schmp. 280-282°C (Zers.)
35. 3,4,5,6,7,8-Hexahydro-7-methyl-3-[3-(4-pyrazin-2-ylpiperazin-1-yl)-propyl]-pyrido[4',3':4,5]thieno[2,3-d]-pyrimidin-4-imin x 4 HCl x H₂O, Schmp. 284-286°C (Zers.)
36. 3,4,5,6,7,8-Hexahydro-7-methyl-3-[3-(4-pyrimidin-2-ylpiperazin-1-yl)-propyl]-pyrido[4',3':4,5]thieno[2,3-d]-pyrimidin-4-imin, Schmp. 161-163°C
37. 3,4,5,6,7,8-Hexahydro-7-methyl-3-[3-(4-(2-cyano-phenyl)-piperazin-1-yl)-propyl]-pyrido[4',3':4,5]thieno[2,3-d]-pyrimidin-4-imin, Schmp. 148-150°C
38. 3,4,5,6,7,8-Hexahydro-7-benzyl-3-[2-(4-(2-methoxy-phenyl)-piperazin-1-yl)-ethyl]-pyrido[4',3':4,5]thieno[2,3-d]-pyrimidin-4-on x 3 HCl x H₂O, Schmp. 288-290°C (Zers.)
39. 3,4,5,6,7,8-Hexahydro-7-methyl-3-[3-(4-(3,4-methylendioxyphenyl)-piperazin-1-yl)-propyl]-pyrido[4',3':4,5]thieno-[2,3-d]pyrimidin-4-imin x 3 HCl, Schmp. 288-290°C (Zers.)
40. 3,4,5,6,7,8-Hexahydro-7-methyl-3-[2-(4-(2-methyl-phenyl)-piperazin-1-yl)-ethyl]-pyrido[4',3':4,5]thieno[2,3-d]-pyrimidin-4-on x 2HCl x H₂O, Schmp. >300°C
41. 3,4,5,6,7,8-Hexahydro-7-methyl-3-[2-(4-(2-chlor-phenyl)-piperazin-1-yl)-ethyl]-pyrido[4',3':4,5]thieno[2,3-d]-pyrimidin-4-on x 2HCl x H₂O, Schmp. >300°C
42. 3,4,5,6,7,8-Hexahydro-7-methyl-3-[2-(4-(3,4-dimethyl-phenyl)-piperazin-1-yl)-ethyl]-pyrido[4',3':4,5]thieno[2,3-d]-pyrimidin-4-on x 2HCl, Schmp. 307-310°C
43. 3,4,5,6,7,8-Hexahydro-7-methyl-3-[2-(4-(2,6-dimethyl-phenyl)-piperazin-1-yl)-ethyl]-pyrido[4',3':4,5]thieno[2,3-d]-pyrimidin-4-on x 2HCl, Schmp. 297-300°C
44. 3,4,5,6,7,8-Hexahydro-7-methyl-3-[2-(4-(2,3-dimethyl-phenyl)-piperazin-1-yl)-ethyl]-pyrido[4',3':4,5]thieno[2,3-d]-pyrimidin-4-on, Schmp. 163-167°C
45. 3,4,5,6,7,8-Hexahydro-7-methyl-3-[2-(4-(2,4-dimethyl-phenyl)-piperazin-1-yl)-ethyl]-pyrido[4',3':4,5]thieno[2,3-d]-pyrimidin-4-on x 2HCl, Schmp. 300-303°C
46. 3,4,5,6,7,8-Hexahydro-7-methyl-3-[2-(4-(3,5-dichlor-phenyl)-piperazin-1-yl)-ethyl]-pyrido[4',3':4,5]thieno[2,3-d]-pyrimidin-4-on, Schmp. 97-100°C
47. 3,4,5,6,7,8-Hexahydro-7-methyl-3-[2-(4-(2,4-dimethoxyphenyl)-piperazin-1-yl)-ethyl]-pyrido[4',3':4,5]thieno-[2,3-d]-pyrimidin-4-on x 2HCl, Schmp. 287-290°C
48. 3,4,5,6,7,8-Hexahydro-7-methyl-3-[2-(4-(3-trifluormethylphenyl)-piperazin-1-yl)-ethyl]-pyrido[4',3':4,5]thieno-[2,3-d]-pyrimidin-4-on x 2HCl, Schmp. 309-312°C
49. 3,4,5,6,7,8-Hexahydro-7-methyl-3-[2-(4-naphth-1-yl-piperazin-1-yl)-ethyl]-pyrido[4',3':4,5]thieno[2,3-d]-pyrimidin-4-on x 2HCl x H₂O, Schmp. 298-300°C (Zers.)
50. 3,4,5,6,7,8-Hexahydro-7-methyl-3-[3-(4-(3-hydroxy-phenyl)-piperazin-1-yl)-propyl]-pyrido[4',3':4,5]thieno[2,3-d]-pyrimidin-4-imin x 2HCl x 2H₂O, Schmp. 182-184°C (Zers.)
51. 3,4,5,6,7,8-Hexahydro-7-methyl-3-[2-(4-(2-methoxy-5-chlorphenyl)-piperazin-1-yl)-ethyl]-pyrido[4',3':4,5]thieno-[2,3-d]-pyrimidin-4-on x 3HCl, Schmp. 170-172°C (Zers.)
52. 3,4,5,6,7,8-Hexahydro-7-methyl-3-[2-(4-(2,5-dimethoxyphenyl)-piperazin-1-yl)-ethyl]-pyrido[4',3':4,5]thieno-[2,3-d]-pyrimidin-4-on x 3HCl x H₂O, Schmp. 176-178°C (Zers.)
53. 3,4,5,6,7,8-Hexahydro-7-methyl-3-[2-(4-12-methoxy-5-phenylphenyl)-piperazin-1-yl)-ethyl]-pyrido[4',3':4,5]thieno-[2,3-d]-pyrimidin-4-on x H₂O, Schmp. 79-80°C
54. 3,4,5,6,7,8-Hexahydro-7-methyl-3-[2-(4-(2-methoxy-phenyl)-3,4-dehydro-piperidin-1-yl)-ethyl]-pyrido[4',3':4,5]thieno-[2,3-d]-pyrimidin-4-on x 2HCl x 2H₂O, Schmp. 182-185°C (Zers.)
55. 3,4,5,6,7,8-Hexahydro-7-methyl-3-[2-(4-(2-hydroxy-phenyl)-piperazin-1-yl)-ethyl]-pyrido[4',3':4,5]thieno[2,3-d]-pyrimidin-4-on x 2HCl x H₂O, Schmp. 281-283°C (Zers.)
56. 3,4,5,6,7,8-Hexahydro-7-methyl-3-[2-(4-(7-methoxy-naphth-1-yl)-piperazin-1-yl)-ethyl]-pyrido[4',3':4,5]thieno-[2,3-d]-pyrimidin-4-on x 2HCl x H₂O, Schmp. 272-274°C (Zers.)
57. 3,4,5,6,7,8-Hexahydro-7-methyl-3-[2-(4-naphth-1-yl-piperazin-1-yl)-ethyl]-pyrido[4',3',:4,5]thieno[2,3-d]-pyrimidin-4-imin x 3HCl, Schmp. 288-289°C (Zers.)
58. 3,4,5,6,7,8-Hexahydro-7-methyl-3-[2-(4-(4,5-methylendioxybenzyl]-piperazin-1-yl)-ethyl]-pyrido[4',3':4,5]thieno-[2,3-d]-pyrimidin-4-imin x 4HCl x 2H₂O, Schmp. 249-251°C (Zers.)
59. 3,4,5,6,7,8-Hexahydro-7-methyl-3-[2-(4-(6-isopropylpyrimidin-4-yl)-piperazin-1-yl)-ethyl]-pyrido[4',3':4,5]-thieno[2,3-d]-pyrimidin-4-imin x 3HCl x 2H₂O, Schmp. 250-253°C (Zers.)
60. 3,4,5,6,7,8-Hexahydro-7-methyl-3-[2-(4-(2-methoxy-naphth1-yl)-piperazin-1-yl)-ethyl]-pyrido[4',3':4,5]thieno[2,3-d]-pyrimidin-4-on x 2HCl x 2H₂O, Schmp. 241-243°C (Zers.)
61. 3,4,5,6,7,8-Hexahydro-7-methyl-3-[2-(4-(2-methoxy-phenyl)-piperidin-1-yl)-ethyl]-pyrido[4',3':4,5]thieno[2,3-d]-pyrimidin-4-on x 2HCl x 2H₂O, Schmp. 299-301°C (Zers.)
62. 3,4,5,6,7,8-Hexahydro-7-methyl-3-[2-(4-(3,4-dimethoxyphenyl)-piperazin-1-yl)-ethyl]-pyrido[4',3':4,5]thieno-[2,3-d]-pyrimidin-4-on, Schmp. 153-154°C
63. 3,4,5,6,7,8-Hexahydro-7-methyl-3-[3-(4-naphth-1-yl-piperazin-1-yl)-propyl]-pyrido[4',3':4,5]thieno[2,3-d]-pyrimidin-4-on x 3HCl x 2H₂O, Schmp. 206-208°C (Zers.)
64. 3,4,5,6,7,8-Hexahydro-7-methyl-3-[3-(4-pyrimidin-2-ylpiperazin-1-yl)-propyl]-pyrido[4',3':4,5]thieno[2,3-d]-pyrimidin-4-on, Schmp. 161-163°C
65. 3,4,5,6,7,8-Hexahydro-7-methyl-3-[2-(4-chinolin-2-ylpiperazin-1-yl)-ethyl]-pyrido[4',3':4,5]thieno[2,3-d]-pyrimidin-4-on, Schmp. 143-145°C
66. 3,4,5,6,7,8-Hexahydro-7-methyl-3-[2-(4-(2-methyl-naphth-1-yl)-ethyl]-pyrido[4',3':4,5]thieno[2,3-d]-pyrimidin-4-on x 2HCl x 2H₂O, Schmp. 295-297°C (Zers.)
67. 3,4,5,6,7,8-Hexahydro-7-methyl-3-[2-(4-(2-methoxy-3,5-dichlor-phenyl)-piperazin-1-yl)-ethyl]-pyrido[4',3':4,5]-thieno[2,3-d]-pyrimidin-4-on x 2HCl x H₂O, Schmp. 264-267°C (Zers.)
68. 3,4,5,6,7,8-Hexahydro-7-methyl-3-[2-(4-(2-cyano-phenyl)-piperazin-1-yl)-ethyl]-pyrido[4',3':4,5]thieno[2,3-d]-pyrimidin-4-on, Schmp. 162-164°C
69. 3,4,5,6,7,8-Hexahydro-7-methyl-3-[2-(4-(2-chlor-phenyl)-piperazin-1-yl)-ethyl]-pyrido[4',3':4,5]thieno[2,3-d]-pyrimidin-4-on, Schmp. 165-167°C
70. 3,4,5,6,7,8-Hexahydro-7-methyl-3-[2-(4-pyridin-2-ylpiperazin-1-yl)-ethyl]-pyrido[4',3':4,5]thieno[2,3-d]-pyrimidin-4-on x 3HCl x 2H₂O, Schmp. 232-234°C (Zers.)
71. 3,4,5,6,7,8-Hexahydro-7-methyl-3-[2-(4-pyridin-4-ylpiperazin-1-yl)-ethyl]-pyrido[4',3':4,5]thieno[2,3-d]-pyrimidin-4-on x 3HCl x 2H₂O, Schmp. 270-272°C (Zers.)
72. 3,4,5,6,7,8-Hexahydro-7-methyl-3-[2-(4-(5-methoxy-pyrimidin-4-yl)-piperazin-1-yl)-ethyl]-pyrido[4',3':4,5]thieno[2,3-d]-pyrimidin-4-on x 3HCl x 4H₂O, Schmp. 266-268°C (Zers.)
73. 3,4,5,6,7,8-Hexahydro-7-methyl-3-[2-(4-naphth-2-yl-piperazin-1-yl)-ethyl]-pyrido[4',3':4,5]thieno[2,3-d]-pyrimidin-4-on, Schmp. 140-141°C
74. 3,4,5,6,7,8-Hexahydro-7-methyl-3-[2-(4-pyrazin-2-ylpiperazin-1-yl)-ethyl]-pyrido[4,3':4,5]thieno[2,3-d]-pyrimidin-4-on x 3HCl x 3H₂O, Schmp. 170-172°C (Zers.)
75. 3,4,5,6,7,8-Hexahydro-7-methyl-3-[2-(4-tetralin-5-ylpiperazin-1-yl)-ethyl]-pyrido[4',3':4,5]thieno[2,3-d]-pyrimidin-4-on x 3HCl x 2H₂O, Schmp. 285-287°C (Zers.)
76. 3,4,5,6,7,8-Hexahydro-7-methyl-3-[2-(4-indan-1-yl-piperazin-1-yl)-ethyl]-pyrido[4',3':4,5]thieno[2,3-d]-pyrimidin-4-on x 3HCl x 2H₂O, Schmp. 300-301°C (Zers.)
77. 3,4,5,6,7,8-Hexahydro-7-methyl-3-[2-(4-(2-methoxy-4-nitro-5-methyl-phenyl)-piperazin-1-yl)-ethyl]-pyrido[4',3':4,5]-thieno[2,3-d]-pyrimidin-4-on x 2HCl x 2H₂O, Schmp. 210-212°C (Zers.)
78. 3,4,5,6,7,8-Hexahydro-7-methyl-3-[2(4-isochinolin-4-ylpiperazin-1-yl)-ethyl]-pyrido[4',3':4,5]thieno[2,3-d]-pyrimidin-4-on x 3HCl x 3H₂O, Schmp. 290-292°C (Zers.)
79. 3,4,5,6,7,8-Hexahydro-7-methyl-3-[2-(4-(2-methoxy-4-chlor-5-methyl-phenyl)-piperazin-1-yl)-ethyl]-pyrido[4',3':4,5]-thieno[2,3-d]-pyrimidin-4-on x 2HCl x 2H₂O, Schmp. 293-294°C (Zers.)
80. 3,4,5,6,7,8-Hexahydro-7-methyl-3-[2-(4-(2,4-dimethoxyphenyl)-piperazin-1-yl)-ethyl]-pyrido[4',3':4,5]thieno-[2,3-d]-pyrimidin-4-on x 3HCl x 3H₂O, Schmp. 290-291°C (Zers.)
81. 3,4,5,6,7,8-Hexahydro-7-methyl-3-[2-(4-chinazolin-4-ylpiperazin-1-yl)-ethyl]-pyrido[4',3':4,5]thieno[2,3-d]-pyrimidin-4-on x 3HCl x 4H₂O, Schmp. 258-260°C (Zers.)
82. 3,4,5,6,7,8-Hexahydro-7-methyl-3-[2-(4-(3-trifluormethyl-4-chlor-phenyl)-piperazin-1-yl)-ethyl]-pyrido[4',3':4,5]thieno-[2,3-d]-pyrimidin-4-on x 2HCl x 3H₂O, Schmp. 311-312°C (Zers.)
83. 3,4,5,6,7,8-Hexahydro-7-(4-chlor-benzyl)-3-[2-(4-(2-methoxyphenyl)-piperazin-1-yl)-ethyl]-pyrido[4',3':4,5]thieno-[2,3-d]pyrimidin-4-on x 3HCl x H₂O, Schmp. 290-292°C (Zers.)
84. 3,4,5,6,7,8-Hexahydro-7-ethyl-3-[2-(4-(2-methoxy-phenyl)-piperazin-1-yl)-ethyl]-pyrido[4',3':4,5]thieno[2,3-d]-pyrimidin-4-on x 3HCl x H₂O, Schmp. 295-297°C (Zers.)
85. 3,4,5,6,7,8-Hexahydro-7-isopropyl-3-[2-(4-(2-methoxyphenyl)-piperazin-1-yl)-ethyl]-pyrido[4',3':4,5]thieno-[2,3-d]pyrimidin-4-on x 3HCl x H₂O, Schmp. 300-302°C (Zers.)
86. 3,4,5,6,7,8-Hexahydro-7-(4-nitro)-benzyl-3-[2-(4-(2-methoxyphenyl)-piperazin-1-yl)-ethyl]-pyrido[4',3':4,5]thieno-[2,3-d]pyrimidin-4-on x 3HCl x H₂O, Schmp. 214-217°C (Zers.)
87. 3,4,5,6,7,8-Hexahydro-7-(4-methoxy)-benzyl-3-[2-(4-(2-methoxy-phenyl)-piperazin-1-yl)-ethyl]-pyrido[4',3':4,5]-thieno[2,3-d]pyrimidin-4-on x 3HCl x H₂O, Schmp. 278-281°C (Zers.)
88. 3,4,5,6,7,8-Hexahydro-7-(2-phenyl)-ethyl-3-[2-(4-(2-methoxyphenyl)-piperazin-1-yl)-ethyl]-pyrido[4',3':4,5]thieno-[2,3-d]pyrimidin-4-on x 3HCl x H₂O, Schmp. 305-306°C (Zers.)
89. 3,4,5,6,7,8-Hexahydro-7-(3-benzoyl)-propyl-3-[2-(4-(2-methoxy-phenyl)-piperazin-1-yl)-ethyl]-pyrido[4',3':4,5]-thieno[2,3-d]pyrimidin-4-on x 3HCl x H₂O, Schmp. 124-126°C (Zers.)
90. 3,4,5,6,7,8-Hexahydro-7-(4-amino)-benzyl-3-[2-(4-(2-methoxyphenyl)-piperazin-1-yl)-ethyl]-pyrido[4',3':4,5]thieno-[2,3-d]pyrimidin-4-on x HCl x 3H₂O, Schmp. 280-282°C (Zers.)
91. 3,4,5,6,7,8-Hexahydro-7-(3-phenyl)-propyl-3-[2-(4-(2-methoxy-phenyl)-piperazin-1-yl)-ethyl]-pyrido[4',3':4,5]-thieno[2,3-d]pyrimidin-4-on x 2HCl x 3H₂O, Schmp. 301-302°C (Zers.)
92. 3,4,5,6,7,8-Hexahydro-7-(3-phenyl)-propyl-3-[2-(4-naphth-1-yl)-piperazin-1-yl)-ethyl]-pyrido[4',3':4,5]thieno[2,3-d]-pyrimidin-4-on x 2HCl x 2H₂O, Schmp. 306-307°C (Zers.)
93. 3,4,5,6,7,8-Hexahydro-7-(2-(4-methoxy)-phenyl)-ethyl-3-[2-(4-naphth-1-yl)-piperazin-1-yl)-ethyl]-pyrido[4',3':4,5]thieno-[2,3-d]pyrimidin-4-on x 2HCl x 3H₂O, Schmp. 306-308°C (Zers.)
94. 3,4,5,6,7,8-Hexahydro-7-(2-(4-chlor)-phenyl)-ethyl-3-[2-(4-naphth-1-yl)-piperazin-1-yl)-ethyl]-pyrido[4',3':4,5]thieno-[2,3-d]pyrimidin-4-on 2 2HCl x 3H₂O, Schmp. 300-303°C (Zers.)
95. 3,4,5,6,7,8-Hexahydro-7-(2-phenyl)-ethyl-3-[2-(4-naphth-1-yl)-piperazin-1-yl)-ethyl]-pyrido[4',3':4,5]thieno[2,3-d]-pyrimidin-4-on x 2HCl x 3H₂O, Schmp. 295-298°C
96. 3,4,5,6,7,8-Hexahydro-7-(2-(4-hydroxy)-phenyl)-ethyl-3-[2-(4-naphth-1-yl]-piperazin-1-yl)-ethyl]-pyrido[4',3':4,5]thieno-[2,3-d]pyrimidin-4-on x 2HCl x 2H₂O, Schmp. 254-256°C
97. 3,4,5,6,7,8-Hexahydro-7-(2-(4-chlor)-phenyl)-ethyl-3-[2-(4-(2-methoxy-phenyl)-piperazin-1-yl)-ethyl]-pyrido[4',3':4,5]-thieno[2,3-d]pyrimidin-4-on x 3HCl x 2H₂O, Schmp. 304-306°C (Zers.)
98. 3,4,5,6,7,8-Hexahydro-7-(2-naphth-1-yl)-ethyl-3-[2-(4-(2-methoxy-phenyl)-piperazin-1-yl)-ethyl]-pyrido[4',3':4,5]-thieno[2,3-d]pyrimidin-4-on x 2HCl x 2H₂O, Schmp. 293-295°C (Zers.)
99. 3,4,5,6,7,8-Hexahydro-7-(2-benzoyl-amino)-ethyl-3-[2-(4-naphth-1-yl)-piperazin-1-yl)-ethyl]-pyrido[4',3':4,5]thieno-[2,3-d]pyrimidin-4-on x 2HCl x 2H₂O, Schmp. 292-294°C (Zers.)
100. 3,4,5,6,7,8-Hexahydro-7-(2-benzoyl-amino)-ethyl-3-[2-(4-(2-methoxy-phenyl)-piperazin-1-yl)-ethyl]-pyrido[4',3':4,5]-thieno[2,3-d]pyrimidin-4-on x 2HCl x 3H₂O, Schmp. 202-204°C (Zers.)
101. 3,4,5,6,7,8-Hexahydro-7-(3-benzoyl-amino)-propyl-3-[2-(4-(2-methoxy-phenyl)-piperazin-1-yl)-ethyl]-pyrido[4',3':4,5]-thieno[2,3-d]pyrimidin-4-on x 3HCl x 2H₂O, Schmp. 182-183°C (Zers.)
102. 3,4,5,6,7,8-Hexahydro-7-(3-benzoyl-amino)-propyl-3-[2-(4-naphth-1-yl)-piperazin-1-yl)-ethyl]-pyrido[4',3':4,5]thieno-[2,3-d]pyrimidin-4-on x 3HCl x H₂O, Schmp. 128-130°C (Zers.)
103. 3,4,5,6,7,8-Hexahydro-7-(4-phenyl)-butyl-3-[2-(4-(2-methoxyphenyl)-piperazin-1-yl)-ethyl]-pyrido[4',3':4,5]thieno-[2,3-d]pyrimidin-4-on x 3HCl x H₂O, Schmp. 311-312°C (Zers.)
104. 3,4,5,6,7,8-Hexahydro-7-(4-phenyl)-butyl-3-[2-(4-naphth-1-yl)-piperazin-1-yl)-ethyl]-pyrido[4',3':4,5]thieno[2,3-d]-pyrimidin-4-on x 3HCl x H₂O, Schmp. 312-314°C (Zers.)
105.3,4,5,6,7,8-Hexahydro-7-(4-methoxy)-benzyl-3-[2-(4-naphth1-yl)-piperazin-1-yl)-ethyl]-pyrido[4',3':4,5]thieno[2,3-d]-pyrimidin-4-on x 3HCl x H₂O, Schmp. 275-277°C (Zers.)
106.3,4,5,6,7,8-Hexahydro-7-(2-(4-methoxy)-phenyl)-ethyl-3-[2-(4-(2-methoxy-phenyl)-piperazin-1-yl)-ethyl]-pyrido-[4',3':4,5]thieno[2,3-d]pyrimidin-4-on x 3HCl x 3H₂O, Schmp. 297-298°C (Zers.)
107. 3,4,5,6,7,8-Hexahydro-7-(2-phenyl)-ethyl-3-[3-(4-naphth-1-yl)-piperazin-1-yl)-propyl]-pyrido[4',3':4,5]thieno[2,3-d]-pyrimidin-4-on, Schmp. 153-155°C
108. 3,4,5,6,7,8-Hexahydro-7-(2-phenyl)-ethyl-3-[2-(4-pyrimidin-2-yl)-piperazin-1-yl)-ethyl)-pyrido[4',3':4,5]thieno[2,3-d]-pyrimidin-4-on x 2HCl x 3H₂O, Schmp. 304-305°C (Zers.)
109. 3,4,5,6,7,8-Hexahydro-7-(2-phenyl)-ethyl-3-[3-(4-pyrimidin-2-yl)-piperazin-1-yl)-propyl)-pyrido[4',3':4,5]thieno[2,3-d]-pyrimidin-4-on x 3HCl x 2H₂O, Schmp. 302-303°C (Zers.)
110. 3,4,5,6,7,8-Hexahydro-7-(3-benzoyl-amino)-propyl-3-[2-(4-pyrimidin-2-yl)-piperazin-1-yl)-ethyl)-pyrido[4',3':4,5]-thieno[2,3-d]pyrimidin-4-on x 3HCl x 3H₂O, Schmp. 125-127°C (Zers.)
111. 3,4,5,6,7,8-Hexahydro-7-(4-phenyl)-butyl-3-[2-(4-pyrimidin-2-yl)-piperazin-1-yl)-ethyl)-pyrido[4',3':4,5]thieno[2,3-d]-pyrimidin-4-on x 3HCl x 3H₂O, Schmp. 317-319°C (Zers.)
112. 3,4,5,6,7,8-Hexahydro-7-(2-(4-methoxy)-phenyl)-ethyl-3-[2-(4-pyrimidin-2-yl)-piperazin-1-yl)-ethyl-pyrido[4',3':4,5]-thieno[2,3-d]pyrimidin-4-on, Schmp. 165-167°C
113. 3,4,5,6,7,8-Hexahydro-7-acetyl-3-[3-(4-(2-methoxy-phenyl)-piperazin-1-yl)-propyl]-pyrido(4',3':4,5]thieno[2,3-d]-pyrimidin-4-imin x 2HCl, Schm. 265-268°C
114. 3,4,5,6,7,8-Hexahydro-7-acetyl-3-[3-(4-(2-methoxy-phenyl)-piperazin-1-yl)-propyl]-pyrido[4',3':9,5]thieno[2,3-d]pyrimid in-4-on x 2HCl x 2H₂O, Schmp. 264-267°C
115. 3,4,5,6,7,8-Hexahydro-3-[2-(4-(2-methoxy-phenyl)-piperazin-1-yl)-ethyl]-pyrido[4',3':4,5]thieno[2,3-d]pyrimidin-4-on, Schmp. 168-170°C
116. 3,4,5,6,7,8-Hexahydro-7-acetyl-3-[2-(4-(2-methoxy-phenyl)-piperazin-1-yl)-ethyl]-pyrido[4',3':4,5]thieno[2,3-d]-pyrimidin-4-on, Schmp. 170-172°C
117. 3,4,5,6,7,8-Hexahydro-7-benzoyl-3-[2-(4-(2-methoxy-phenyl)-piperazin-1-yl)-ethyl]-pyrido[4',3':4,5]thieno[2,3-d]-pyrimidin-4-on x 2HCl x 2H₂O, Schmp. 185-187°C (Zers.)
118. 3,4,5,6,7,8-Hexahydro-7-benzoyl-3-[2-(4-naphth-1-yl)-piperazin-1-yl)-ethyl]-pyrido[4',3':4,5]thieno[2,3-d]-pyrimidin-4-on, Schmp. 195-197°C
119.3,4,5,6,7,8-Hexahydro-7-benzoyl-3-[2-(4-pyrimidin-2-yl)-piperazin-1-yl)-ethyl]-pyrido[4',3':4,5]thieno[2,3-d]-pyrimidin-4-on, Schmp. 130-132°C (Zers.)
120. 3,4,5,6,7,8-Hexahydro-2,7-dimethyl-3-[2-(4-(2-methoxyphenyl)-piperazin-1-yl)-ethyl]-pyrido[4',3':4,5]thieno-[2,3-d]pyrimidin-4-on, Schmp. 176-178°C
121.3,4,5,6,7,8-Hexahydro-7-acetyl-2-hydroxy-3-[2-(4-(2-methoxyphenyl]-piperazin-1-yl)-ethyl]-pyrido[4',3':4,5]thieno-[2,3-d]pyrimidin-4-on, Schmp. 135-137°C
122. 3,4,5,6,7,8-Hexahydro-7-methyl-3-[1-(4-(2-methoxy-phenyl)-piperazin-1-yl)-prop-2-yl]-pyrido[4',3':4,5]thieno[2,3-d]-pyrimidin-4-on, Schmp. 184-186°C
123. 3,4,5,6,7,8-Hexahydro-[1-(4-naphth-1-yl-piperazin-1-yl)-prop-2-yl]-pyrido[4',3':4,5]thieno[2,3-d]pyrimidin-4-on x 2HCl x 4H₂O, Schmp. 242-244°C (Zers.)
124. 3,4,5,6,7,8-Hexahydro-7-methyl-3-[2-(4-(2-methoxy-phenyl)-piperazin-1-yl)-propyl]-pyrido[4',3':4,5]thieno[2,3-d]-pyrimidin-4-on x 3HCl x 3H₂O, Schmp. 190-192°C (Zers.)

### C Messung der Rezeptorbindung

### Präparation der rezeptortragenden Zellmembranen

Die Rezeptorbindungsstudien wurden mit "Membranpräparationen" durchgeführt, die aus Zellkulturen der menschlichen embryonalen Nierenzellinie 293 (HEK 293) gewonnen wurden, in die jeweils ein spezifischer Serotoninrezeptor-Subtyp (h5HT1A, h5HT1B oder h5HT1D) einkloniert ist und permanent exprimiert wird.

Das Wachstum der Zellen erfolgte im RPMI 1640 Medium (Life Technologies), das zusätzlich 10 % fötales Kälberserum (PCS), 2 mmol/l L-Glutamin und 400 mg/l Geneticin G 418 enthielt. Die Zellen wurden bis zum Erreichen einer geschlossenen, einschichtigen Zellschicht ("Monolayer") in sog. "Wannenstapel" in einem Luft/5 % CO₂ begasten Brutschrank bei 37°C inkubiert. Die Zellen wurden anschließend unter Verwendung eines Puffers folgender Zusammensetzung von den Kulturgefäßen abgelöst: (Angaben pro Liter) Trypsin 10 mg; EDTA 4 mg; EGTA 200 mg; KCl 200 mg; KH₂PO₄ 200 mg; Na₂HPO₄ 1,15 g; NaCl 8,0 g; pH 7,4. Die Zellsuspension wurde pelletiert in Dulbecco's phosphatgepufferte Saline (PBS) resuspendiert und die Zelldichte auf ca. 10⁸ Zellen/ml eingestellt. Nach erneutem Pelletieren wurde PBS durch dasselbe Volumen eiskalten Lysispuffer (5 mmol/l Tris; 10 % Glyzerin; pH 7,4) ersetzt und 30 Min. bei 4°C inkubiert. Die lysierten Zellen (= "Membranen") wurden aliquotiert und bis zum Einsatz in Rezeptorbindungsstudien in flüssigem Stickstoff gelagert. Ein Aliquot pro Präparation fand zur Bestimmung des Proteingehaltes Verwendung.

Die erfindungsgemäßen Verbindungen zeigen eine hohe Affinität (K; ≦ 30 nM) zu humanen 5-HT_{1A}, 5-HT_{1B} und 5-HT_{1D} Rezeptortypen, die in klonierten Zellinien exprimiert sind.

### Rezeptorbindungsassay

Die Rezeptorbindungsstudien wurden in 1 ml Makrowell-Röhrchen durchgeführt, die folgende Komponenten enthielten:
- 50 µl der Testsubstanz in verschiedenen Konzentrationen für Kompetitionsmessungen oder 50 µl Assay-Puffer oder 50 µl nicht-markiertes Serotonin (1 µmol/l final) zur Bestimmung der totalen bzw. unspezifischen Bindungskontrolle
- 200 µl Membransuspension des entsprechenden Rezeptorsubtyps mit einem Proteingehalt von 200 µg/Röhrchen
- 250 µl Radioligandenlösung ([³H]5-Carboxamidotryptamin (5-CT) für h5HT1B- und h5HT1D-Rezeptoren oder [³H]8-Hydroxy-dipropylaminotetralin (8-OH-DPAT) für h5HT1A-Rezeptoren. Die finalen Konzentrationen der Radioliganden wurden auf 3 nmol/l bzw. 0,3 nmol/l eingestellt.

Der Assay-Puffer (pH 7,4) hatte folgende Zusammensetzung (pro Liter): Tris 6,057 g; CaCl₂x2H₂O = 5,88 g; Ascorbinsäure 1 g; Pargylin 1,96 mg.

Der Assay-Ansatz wurde 30 Min. bei 25°C inkubiert und anschließend über Fiberglas-Filter (Whatman GF/B) mittels eines Zellerntegerätes (Skatron) filtriert und die Filter mit 5 bis 9 ml kaltem Puffer gewaschen. Die Filter wurden in Szintillationsgläschen mit jeweils 5 ml Ultima GoldxR Flüssigszintillator (Packard) vereinigt, 1 Stunde geschüttelt und anschließend wurde die Radioaktivität in einem Beta-Zählgerät (Wallac) bestimmt. Die Meßdaten wurden mittels einer iterativen nicht-linearen Regressionsanalyse mit dem "Statistical Analysis System (SAS), das dem von Munson und Rodbard (Anal. Biochem: 107, 220 (1980)) beschriebenen "LIGAND"-Programm ähnlich ist, ausgewertet. Die Kompetitionskonstanten (Kᵢ) wurden in nmol/l angegeben.

## Patentansprüche

1. 3-substituiertes 3,4,5,6,7,8-Hexahydro-pyrido [4',3':4,5] thieno [2,3-d] pyrimidinDerivat der Formel I 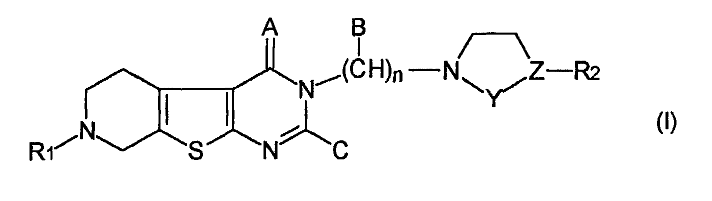 worin
R¹ ein Wasserstoffatom, eine C₁-C₄-Alkylgruppe, eine Acetyl- oder Benzoylgruppe, ein Phenylalkyl C₁-C₄ Rest, wobei der Aromat gegebenenfalls durch Halogen, C₁-C₄-Alkyl-, Trifluormethyl-, Hydroxy-, C₁-C₄-Alkoxy-, Amino-, Cyano- oder Nitrogruppen substituiert ist, ein Naphthylalkyl C₁-C₃-Rest, ein Phenylalkanon C₂-C₃-Rest oder ein Phenylcarbamoylalkyl C₂-Rest bedeutet, wobei die Phenylgruppe durch Halogen substituiert sein kann,
R² eine gegebenenfalls durch Halogenatome, C₁-C₄-Alkyl, Trifluormethyl-, Trifluormethoxy-, Hydroxy-, C₁-C₄-Alkoxy-, Amino-, Monomethylamino-, Dimethylamino-, Cyano- oder Nitrogruppen mono-, di- oder trisubstituierte Phenyl-, Pyridyl-, Pyrimidinyl- oder Pyrazinyl-Gruppe darstellt, die gegebenenfalls mit einem Benzolkern, der gegebenenfalls durch Halogenatome, C₁-C₄-Alkyl, Hydroxy-, Trifluormethyl, C₁-C₄-Alkoxy-, Amino-, Cyano- oder Nitrogruppen mono- oder disubstituiert sein kann und gegebenenfalls 1 Stickstoffatom enthalten kann, oder mit einem 5- oder 6-gliedrigen Ring, der 1-2 Sauerstoffatome enthalten kann, anelliert sein kann,
oder durch eine Phenyl-C₁-C₂-alkyl-bzw. -alkoxy-Gruppe substituiert sein kann, wobei der Phenylrest durch Halogen, eine Methyl-, Trifluormethyl- oder Methoxygruppe substituiert sein kann,
A NH oder ein Sauerstoffatom darstellt,
B Wasserstoff oder Methyl bedeutet,
C Wasserstoff, Methyl oder Hydroxy darstellt,
Y CH₂, CH₂-CH₂, CH₂-CH₂-CH₂ oder CH₂-CH ist,
Z ein Stickstoffatom, Kohlenstoffatom oder CH darstellt, wobei die Bindung zwischen Y und Z auch eine Doppelbindung sein kann,
und n die Zahl 2, 3 oder 4 bedeutet,
sowie deren physiologisch verträgliche Salze.

2. Verbindung nach Anspruch 1, **dadurch gekennzeichnet, dass**
R¹ Methyl, Ethyl, Isopropyl, Benzyl, subst. Benzyl, Phenethyl, subst. Phenethyl,
R² o-Methoxyphenyl, 1-Naphthyl, Pyrimidin-2-yl, 2-Methoxy-1-naphthyl, 2-Methyl-1-naphthyl,
A NH oder ein Sauerstoffatom,
Y CH₂-CH₂, CH₂-CH,
Z ein Stickstoffatom, Kohlenstoffatom oder CH
und n die Zahl 2 oder 3 bedeuten.

3. Verbindung nach Anspruch 1 oder 2 zur Verwendung als Arzneimittel.

4. Verwendung einer Verbindung nach Anspruch 1 oder 2 zur Herstellung eines Arzneimittels zur Behandlung von Depressionen und verwandten Krankheiten.

## Claims

1. 3-substituted 3,4,5,6,7,8-hexahydropyrido[4',3':4,5]-thieno[2,3-d]pyrimidine derivative of the Formula I 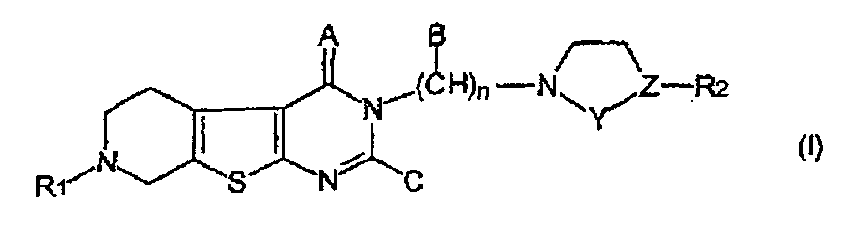 wherein
R¹ denotes a hydrogen atom, a C₁-C₄-alkyl group, an acetyl or benzoyl group, a phenylalkyl C₁-C₄ radical, wherein the aromatic radical may optionally be substituted by halogen, C₁-C₄-alkyl, trifluoromethyl, hydroxy, C₁-C₄-alkoxy, amino, cyano or nitro groups, a naphthylalkyl C₁-C₃ radical, a phenylalkanone C₂-C₃ radical or a phenylcarbamoylalkyl C₂ radical, wherein the phenyl group may be substituted by halogen,
R² denotes a phenyl, pyridyl, pyrimidinyl or pyrazinyl group optionally monosubstituted, disubstituted or trisubstituted by halogen atoms, C₁-C₄-alkyl, trifluoromethyl, trifluoromethoxy, hydroxy, C₁-C₄-alkoxy, amino, monoethylamino, dimethylamino, cyano or nitro groups, which may optionally be monosubstituted or disubstituted with a benzene nucleus that may optionally be substituted by halogen atoms, C₁-C₄-alkyl, hydroxy, trifluoromethyl, C₁-C₄-alkoxy, amino, cyano or nitro groups and may optionally contain one nitrogen atom, or may be fused to a 5-membered or 6-membered ring that may contain 1-2 oxygen atoms, or may be substituted by a phenyl-C₁-C₂-alkyl or phenyl-C₁-C₂-alkoxy group, wherein the phenyl radical may be substituted by halogen, a methyl, trifluoro-methyl or methoxy group,
A denotes NH or an oxygen atom,
B denotes hydrogen or methyl
C denotes hydrogen, methyl or hydroxy,
Y is CH₂, CH₂-CH₂, CH₂-CH₂-CH₂ or CH₂-CH,
Z denotes a nitrogen atom, carbon atom or CH, wherein the bond between Y and Z may also be a double bond,
and n denotes the number 2, 3 or 4,
as well as their physiologically compatible salts.

2. Compound according to claim 1, **characterised in that**
R¹ denotes methyl, ethyl, isopropyl, benzyl, substituted benzyl, phenethyl, substituted phenethyl,
R² denotes o-methoxyphenyl, 1-naphthyl, pyrimidin-2-yl, 2-methoxy-1-naphthyl, 2-methyl-1-naphthyl,
A denotes NH or an oxygen atom,
Y is CH₂-CH₂, CH₂-CH,
Z denotes a nitrogen atom, carbon atom or CH
and n denotes the number 2 or 3.

3. Compound according to claim 1 or 2 for use as a medicament.

4. Use of a compound according to claim 1 or 2 for the production of a medicament for treating depression and related disorders.

## Revendications

1. Dérivé de 3,4,5,6,7,8-hexahydro-pyrido[4',3':4,5]thiéno[2,3-d]pyrimidine 3-substitué de formule I 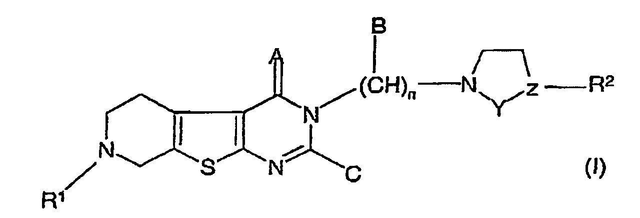 où
R¹ représente un atome d'hydrogène, un groupe alkyle en C₁-C₄, un groupe acétyle ou benzoyle, un reste phényl-alkyle en C₁-C₄ où le groupe aromatique est éventuellement substitué par des groupes halogène, alkyle en C₁-C₄, trifluorométhyle, hydroxyle, alcoxy en C₁-C₄, amino, cyano ou nitro, un reste naphtyl-alkyle en C₁-C₃, un reste phénylalcanone en C₂-C₃ ou un reste phénylcarbamoyl-alkyle en C₂, où le groupe phényle peut être substitué par halogène,
R² représente un groupe phényle, pyridyle, pyrimidinyle ou pyrazinyle éventuellement mono-, di- ou tri-substitué par des atomes d'halogène, des groupes alkyle en C₁-C₄, trifluorométhyle, trifluorométhoxy, hydroxyle, alcoxy en C₁-C₄, amino, monoéthylamino, diméthylamino, cyano ou nitro, qui peut éventuellement être substitué avec un noyau benzénique, qui peut éventuellement être mono- ou di-substitué par des atomes d'halogène, des groupes alkyle en C₁-C₄, hydroxyle, trifluorométhyle, alcoxy en C₁-C₄, amino, cyano ou nitro et qui peut éventuellement contenir un atome d'azote, ou être fusionné avec un cycle à 5 ou 6 chaînons qui peut contenir 1-2 atomes d'oxygène,
ou qui peut être substitué par un groupe phényl-alkyle ou alcoxy en C₁-C₂, où le reste phényle peut être substitué par halogène, un groupe méthyle, trilfuorométhyle ou méthoxy,
A représente NH ou un atome d'oxygène,
B représente l'hydrogène ou méthyle,
C représente l'hydrogène, méthyle ou hydroxyle,
Y est CH₂, CH₂-CH₂, CH₂-CH₂-CH₂ ou CH₂-CH,
Z représente un atome d'azote, un atome de carbone ou CH, où la liaison entre Y et Z peut aussi être une double liaison,
et n représente le nombre 2, 3 ou 4,
ainsi que ses sels physiologiquement acceptables.

2. Composé selon la revendication 1, **caractérisé en ce que**
R¹ représente méthyle, éthyle, isopropyle, benzyle, benzyle substitué, phénéthyle, phénéthyle substitué,
R² représente o-méthoxyphényle, 1-naphtyle, pyrimidin-2-yle, 2-méthoxy-1-naphtyle, 2-méthyl-1-naphtyle,
A représente NH ou un atome d'oxygène,
Y représente CH₂-CH₂, CH₂-CH,
Z représente un atome d'azote, un atome de carbone ou CH et
n représente le nombre 2 ou 3.

3. Composé selon la revendication 1 ou 2 destiné à être utilisé comme médicament.

4. Utilisation d'un composé selon la revendication 1 ou 2 pour la production d'un médicament pour le traitement des dépressions et des maladies apparentées.
